# FASCICULE DE BREVET EUROPEEN

(11) **EP 2 435 824 B1**
(45) Date de publication et mention de la délivrance du brevet: **14.05.2014**
(21) Numéro de dépôt: 10724410.5
(22) Date de dépôt: 28.05.2010
(51) Int. Cl.: G01N 33/18, G01N 31/22

(54) **RÉACTIF CHIMIQUE POUR LA MESURE DU TAUX D'AGENTS HALOGÈNES NOTAMMENT DANS DES EAUX DE PISCINE AINSI QU'UN PROCÉDÉ DE MESURE ASSOCIÉ**
CHEMISCHES REAGENS ZUR MESSUNG DER KONZENTRATION VON HALOGENSTOFFEN, INSBESONDERE IN SCHWIMMBADWASSER, UND DAMIT VERBUNDENES MESSVERFAHREN
CHEMICAL REAGENT FOR MEASURING THE LEVEL OF HALOGEN AGENTS, IN PARTICULAR IN SWIMMING-POOL WATER AND ASSOCIATED MEASUREMENT METHOD

(30) Priorité: 28.05.2009 FR 0902580
(43) Date de publication de la demande: 04.04.2012
(73) Titulaire: Pacific Industrie, 24750 Boulazac (FR)
(72) Inventeur: STADNICKI, Zbigniew, F-24150 Cales (FR)
(74) Mandataire: Parzy, Benjamin Alain
(86) Numéro de dépôt international: PCT/EP2010/057496
(87) Numéro de publication internationale: WO 2010/136595

(56) Documents cités:
- WO-A1-97/12242
- WO-A1-2007/044154
- DE-B- 1 001 507
- FR-A1- 2 476 844
- US-A- 4 938 926
- US-A1- 2006 073 603
- DATABASE WPI Week 198910 Thomson Scientific, London, GB; AN 1989-072750 XP002556040 & JP 1 025042 A (TATSUTA DENSEN KK) 27 janvier 1989 (1989-01-27)

## Description

La présente invention concerne un réactif chimique pour la mesure du taux d'agents halogénés notamment dans des eaux de piscine ainsi qu'un procédé de mesure associé.

L'invention trouvera avantageusement application dans le domaine de la filtration et du contrôle des eaux de piscine permettant le suivi de la concentration en chlore ou en brome des eaux de piscines résidentielles.

Toutefois bien que particulièrement prévus pour une telle application le réactif chimique et le procédé associé pourront être utilisés dans le contrôle et le traitement d'eau de bassins collectifs notamment dans les stations thermales ou encore de bassins de type variés et notamment de jacuzzi ou encore d'aquarium.

### ARRIERE PLAN DE L'INVENTION

Dans le domaine de la filtration et du contrôle des eaux de piscine, on réalise régulièrement un contrôle de la concentration en désinfectant de manière à d'une part éviter tout risque sanitaire et d'autre part à optimiser la consommation en désinfectant halogéné.

A cet effet on utilise différents réactifs chimiques permettant la mise en oeuvre de procédés manuels utilisant des bandelettes imbibées de réactif à tremper dans l'eau ou encore de procédés automatiques, dans lesquels le réactif est mélangé avec les eaux de piscine puis l'ensemble introduit dans une chambre d'analyse pour réaliser différents types de mesure et notamment des mesures colorimétriques.

L'art antérieur est notamment illustré par le document WO97/12242 A1.

La présente invention se situe dans le domaine de la mesure automatique du taux d'agents halogénés permettant de déterminer sans intervention manuelle la concentration d'agents halogénés tels que le chlore ou le brome.

Jusqu' à présent le réactif chimique couramment utilisé dans la mesure du taux d'agents halogénés, à savoir le DDP est transparent et ne prend une teinte particulière que lorsqu'il réagit avec l'eau si toutefois cette eau comprend une concentration suffisante d'agents halogénés. Un inconvénient du réactif utilisé réside dans le fait que les procédés actuels basés sur une mesure optique ne peuvent différentier le cas où l'eau à analyser ne contient pas d'agents halogénés du cas ou le réactif chimique n'est pas mélangé dans l'eau à analyser. En effet dans les deux cas le mélange ne varie pas optiquement et reste transparent. Cette impossibilité de discriminer les deux cas précités est problématique puisqu'en cas de non détection de réaction les procédés actuels en déduisent une concentration en agents halogénés nulle alors même que cette concentration peut être supérieure au seuil autorisé.

Par ailleurs, l'emploi du DDP comme réactif chimique est problématique car ce réactif présente une toxicité importante. En effet, ce réactif est classé comme toxique dans la directive REACH et par conséquent ne pourra plus être utilisé sur le marché européen ; il y a donc un intérêt important à proposer un réactif chimique avec une nouvelle formulation pouvant être utilisée sans risque de toxicité.

### OBJET DE L'INVENTION

La présente invention a pour objet de pallier le manque de réactif non toxique pour la réalisation de mesure selon des procédés automatiques et de proposer un réactif chimique permettant de déterminer de façon rapide et précise la concentration en agents halogénés d'une solution, sans risque de fausse mesure du à une absence de réactif.

### RESUME DE L'INVENTION

A cet effet la présente invention concerne un réactif chimique, pour la mesure par absorbance du taux d'agents halogénés d'une solution notamment dans des eaux de piscine comprenant, selon l'invention, un polysaccharide, un composé stabilisant acide, de l'iodure de potassium, un colorant et de l'eau distillé.

De façon préférée, le réactif de l'invention comporte également un composé basique, notamment de la soude.

### BREVE DESCRIPTION DES DESSINS

La présente invention sera mieux comprise à la lecture d'un exemple détaillé de réalisation en référence au dessin annexé, fourni à titre d'exemple non limitatif, représentant la courbe d'absorption du mélange eau/réactif en fonction de la concentration en chlore de l'eau.

### DESCRIPTION DETAILLEE DE L'INVENTION

Le réactif chimique selon un mode particulier de mise en oeuvre de l'invention comprend les éléments suivants :
- un polysaccharide,
- un composé stabilisant acide,
- de l'iodure de potassium
- de la soude,
- un colorant,
- de l'eau distillée.

De préférence le colorant est de l'azorubine, ce colorant rouge, non toxique, est également connu comme colorant alimentaire sous la référence E122. D'autres colorants, de couleurs rouge ou autres, sont toutefois bien entendu également envisageables.

Le colorant permet de détecter la présence du réactif chimique dans le mélange et par conséquent de pouvoir discriminer le cas où l'eau ne contient pas d'agents halogénés, et donc ne réagit pas à la présence du réactif chimique, du cas où le réactif chimique n'a pas été injecté dans le mélange.

A cette fin le procédé comporte une étape de vérification colorimétrique du mélange.

Avantageusement le polysaccharide est fourni sous forme d'amidon de pomme de terre.

Le stabilisant acide est de l'acide chlorhydrique, toutefois d'autres acides sont également envisageables.

Avantageusement le réactif chimique comprend :
- 8 à 10 % de polysaccharide,
- 2 à 3 % d'acide chlorhydrique,
- 2.5 à 3.5% d'iodure de potassium,
- 2 à 3% de soude,
- 0.5 à 1.5 % de colorant.

La préparation du réactif chimique s'effectue en plusieurs étapes. Dans la première étape on réalise l'hydrolyse à chaud du polysaccharide, du composé stabilisant acide et de l'iodure de potassium.

Dans une seconde étape le résultat de l'étape d'hydrolyse est refroidi à température ambiante. Enfin dans une troisième étape on ajoute, à l'issue de la seconde étape la soude, le colorant et de l'eau distillée.

Selon un mode de réalisation préféré de l'invention, le réactif chimique comprend par litre de réactif :
- 91g d'amidon de pomme de terre à 20%
- 23ml d'acide chlorhydrique
- 30g d'iodure de potassium
- 23ml de soude
- 10ml de colorant
- 864ml d'eau distillée.

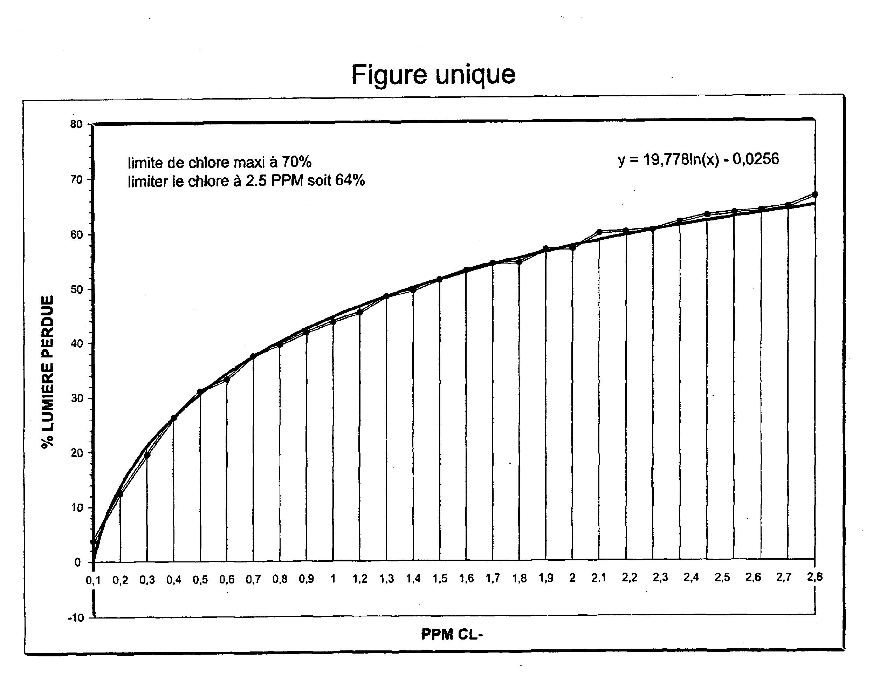

Cette composition est particulièrement adaptée pour la mesure des eaux de piscine, en effet en se reportant à la figure 1 on voit représentée une courbe de référence de l'absorption de la lumière par le mélange eau/réactif en fonction de la concentration en chlore dans l'eau. Le graphique reprend en abscisse la quantité de chlore en partie par million (PPM) et en ordonnée le taux d'absorbance d'un mélange eau/réactif.

Le réactif est très bien adapté car il permet une mesure précise pour des concentrations en chlore allant de 0.1 à 2.8 ppm, la quantité de chlore dans un bassin devant être généralement inférieure à 2.5 ppm. En effet, une faible variation de la concentration dans cette gamme de 0.1 à 2.8 ppm entraine une modification aisément détectable par des appareillages classiques du taux d'absorption du mélange. A titre d'exemple une chute de la concentration du chlore de 2.5ppm à 1ppm entraîne une variation du taux d'absorption de 20 points passant de 64% à 44%.

Par ailleurs ce réactif présente une grande réactivité et l'analyse de l'eau peut être effectuée quelques secondes après introduction du mélange eau/réactif dans la chambre d'analyse.

Le procédé de mesure utilisé avec le réactif chimique décrit ci-dessus comporte une étape de mise en contact du réactif avec un volume d'eau à analyser, une étape de vérification de la présence du réactif, une étape de mesure d'absorbance du mélange et une étape de comparaison de la mesure obtenue dans l'étape précédente, avec une courbe de référence, de manière à déterminer le taux d'halogènes dans l'eau de piscine.

La première étape est réalisée de préférence en introduisant un volume d'eau de la piscine dans une voie d'un mélangeur à trois voies et en injectant parallèlement une dose de réactif par une seconde voie du mélangeur.

Le mélange sort du mélangeur par la troisième voie est ensuite injecté dans une chambre d'analyse permettant de procéder à la seconde étape dite de vérification. Dans cette étape un capteur optique vérifie la présence de la couleur du colorant dans le mélange et permet d'éviter une erreur de mesure, due à une absence de réactif, dans la troisième étape.

La troisième étape dite de mesure consiste, avantageusement cette seconde étape consiste à éclairer la chambre d'analyse et à réaliser la mesure de la lumière absorbée par le mélange.

Cette mesure obtenue, le procédé consiste ensuite, dans une quatrième étape à comparer cette valeur a une courbe de référence préétablie telle que représentée à la figure 1 pour l'analyse des eaux chlorées.

La mesure de l'absorbance obtenue dans l'étape précédente et la relation entre l'absorbance du mélange et la concentration de chlore, telle qu'illustrée à la figure 1, permettent de déterminer la concentration du mélange.

Une fois le taux de chlore déterminé, le procédé peut comprendre des étapes supplémentaires permettant de commander un distributeur d'agent désinfectant afin de réguler automatiquement, en fonction de la concentration en temps réel de chlore, la quantité d'agent désinfectant à verser dans la piscine.

Une autre étape supplémentaire consiste à effectuer des mesures complémentaires sur le même échantillon ou sur un nouvel échantillon éventuellement en modifiant le dosage entre l'eau à analyser et le réactif chimique.

D'autres caractéristiques de l'invention auraient également pu être envisagées sans pour autant sortir du cadre de l'invention définie par les revendications ci-après.

A titre d'exemple les deuxième et troisième étapes du procédé seront, dans une variante, réalisées, simultanément.

Bien que dans l'exemple de mise en oeuvre détaillé ici, on ait indiqué que le réactif contenait un composé basique, en l'occurrence de la soude, qui rend le réactif particulièrement adapté aux eaux de piscine, on pourra se passer de ce composé, ou utiliser un autre composé basique.

## Revendications

1. Réactif chimique pour la mesure par absorbance du taux d'agents halogénés d'une solution notamment dans des eaux de piscine, comprenant un polysaccharide, un composé stabilisant acide, de l'iodure de potassium et de l'eau distillée, **caractérisé en ce que** le réactif comporte en outre un colorant.

2. Réactif chimique selon la revendication 1 dans lequel le colorant est de l'azorubine.

3. Réactif chimique selon la revendication 1 dans lequel le polysaccharide est fourni sous forme d'amidon de pomme de terre.

4. Réactif chimique selon la revendication 1 dans lequel le stabilisant acide est de l'acide chlorhydrique.

5. Réactif chimique selon la revendication 1, comportant en outre un composé basique, notamment de la soude.

6. Réactif chimique selon la revendication 1 dans lequel le réactif chimique comprend :
- 8 à 10 % de polysaccharide
- 2 à 3 % d'acide chlorhydrique
- 2.5 à 3.5% d'iodure de potassium
- 2 à 3% de soude
- 0.5 à 1.5 % de colorant.

7. Réactif chimique selon la revendication 1 dans lequel le réactif chimique comprend par litre de réactif :
- 91g d'amidon de pomme de terre à 20%
- 23ml d'acide chlorhydrique
- 30g d'iodure de potassium
- 23ml de soude
- 10ml de colorant
- 864ml d'eau distillée.

8. Procédé pour la mesure automatique du taux d'agents halogénés d'une solution notamment dans des eaux de piscine utilisant un réactif chimique selon l'une quelconque des revendications précédentes comportant :
- une étape de mise en contact du réactif avec un volume d'eau à analyser,
- une étape de vérification de la présence du réactif dans le mélange,
- une étape de mesure d'absorbance du mélange,
- une étape de comparaison de la mesure obtenue dans l'étape précédente avec une courbe de référence de manière à déterminer le taux d'halogènes dans l'eau de piscine.

## Patentansprüche

1. Chemisches Reagens zur Absorptionsmessung der Konzentration von Halogenstoffen in einer Lösung, insbesondere im Schwimmbadwasser, umfassend ein Polysaccharid, eine sauren Stabilisator, Kaliumjodid und destilliertes Wasser, **dadurch gekennzeichnet, dass** das Reagens außerdem einen Farbstoff enthält.

2. Chemisches Reagens nach Anspruch 1, wobei der Farbstoff Azorubin ist.

3. Chemisches Reagens nach Anspruch 1, wobei das Polysaccharid in Form von Kartoffelstärke bereitgestellt ist.

4. Chemisches Reagens nach Anspruch 1, wobei der saure Stabilisator Salzsäure ist.

5. Chemisches Reagens nach Anspruch 1, des Weiteren umfassend eine basische Verbindung, insbesondere Natron.

6. Chemisches Reagens nach Anspruch 1, wobei das chemische Reagens umfasst:
- 8 bis 10% Polysaccharid
- 2 bis 3% Salzsäure
- 2,5 bis 3,5% Kaliumjodid
- 2 bis 3% Natron
- 0,5 bis 1,5% Farbstoff.

7. Chemisches Reagens nach Anspruch 1, wobei das chemische Reagens pro Liter Reagens umfasst:
- 91 g 20%ige Kartoffelstärke
- 23ml Salzsäure
- 30g Kaliumjodid
- 23ml Natron
- 10ml Farbstoff
- 864ml destilliertes Wasser.

8. Verfahren zur automatischen Messung der Konzentration von Halogenstoffen in einer Lösung, insbesondere im Schwimmbadwasser, unter Verwendung eines chemischen Reagens nach einem der vorhergehenden Ansprüche, umfassend die folgenden Schritte:
- das Reagens mit einer zu analysierenden Wassermenge in Kontakt bringen,
- das Vorhandensein des Reagens in der Mischung verifizieren,
- Absorptionsmessung der Mischung,
- Vergleichen der im vorherigen Schritt erhaltenen Messung mit einer Referenzkurve derart, dass die Halogenkonzentration im Schwimmbadwasser bestimmt wird.

## Claims

1. A chemical reagent for absorbance measuring the halogen-agent content of a solution in particular in swimming pool water, the reagent being **characterized in that** it comprises a polysaccharide, an acid stabilizer compound, potassium iodide, and distilled water **characterized in that** the reagent comprises a dye.

2. A chemical reagent according to claim 1, wherein the dye is azorubine.

3. A chemical reagent according to claim 1, wherein the polysaccharide is provided in the form of potato starch.

4. A chemical reagent according to claim 1, wherein the acid stabilizer is hydrochloric acid.

5. A chemical reagent according to claim 1, further including a basic compound, in particular caustic soda.

6. A chemical reagent according to claim 1, wherein the chemical reagent includes:
· 8% to 10% polysaccharide;
· 2% to 3% hydrochloric acid;
· 2.5% to 3.5% potassium iodide;
· 2% to 3% caustic soda; and
· 0.5% to 1.5% dye.

7. A chemical reagent according to claim 1, wherein the chemical reagent comprises, per liter of reagent:
· 91 g of 20% potato starch;
· 23 mL of hydrochloric acid;
· 30 g of potassium iodide;
· 23 mL of caustic soda;
· 10 mL of dye; and
· 864 mL of distilled water.

8. A method of measuring the content of halogen agents of solution in particular in swimming pool water by using a chemical reagent according to any preceding claim, the method comprising:
· a step of putting the reagent into contact with a volume of water for analysis;
· a step of verifying the presence of the reagent in the mixture;
· a step of measuring the absorbence of the mixture; and
· a step of comparing the measurement obtained in the preceding step with a reference curve so as to determine the halogen content in the swimming pool water.
